# EUROPÄISCHE PATENTANMELDUNG

(11) **EP 1 837 050 A1**
(43) Veröffentlichungstag der Anmeldung: **26.09.2007**
(21) Anmeldenummer: 06006140.5
(22) Anmeldetag: 24.03.2006
(51) Int. Cl.: A61N 5/10, A61N 5/06

(54) **Vorrichtung für die Bestrahlung von Haut**

(71) Anmelder: WaveLight AG, 91058 Erlangen (DE)
(72) Erfinder: Haberer, Daniel, 91301 Forchheim (DE); Kaukel, Sebastian, 91334 Hemhofen (DE)
(74) Vertreter: von Hellfeld, Axel

(57) **Zusammenfassung**

Eine Vorrichtung für die Bestrahlung von Haut weist Einrichtungen (16, 16') auf, mit denen feststellbar ist, ob ein der Haut zugekehrtes Bauteil der Vorrichtung einen vorgegebenen Mindestabstand von der Haut hat. Dann, wenn die Vorrichtung (10) nahe genug an der Haut (12) ist, wird die Abgabe von Behandlungsstrahlung freigegeben.

## Beschreibung

Die Erfindung betrifft eine Vorrichtung für die Bestrahlung von insbesondere Haut.

Der Stand der Technik kennt eine Vielzahl von Geräten für die medizinische und/oder kosmetische Behandlung von insbesondere Haut mit elektromagnetischer Strahlung. Bekannt sind z.B. Geräte für die Lichttherapie mit Laser, LED, oder auch IPL-Systeme. Nachfolgend wird der Begriff "Strahlung" im Sinne von elektromagnetischer Strahlung unterschiedlichster Wellenlängen verstanden, einschließlich IR-, optischer (sichtbarer) und UV-Strahlung.

Geräte dieser Art weisen herkömmlicherweise einen mit dem Fuß oder der Hand betätigbaren Schalter auf, nach dessen Einschalten sich das Gerät im Dauerbetrieb befindet, d.h. die Strahlung wird abgegeben, unabhängig davon, ob das Gerät in Bezug auf die zu bestrahlende Haut eine bestimmte Stellung einnimmt oder nicht. Dies kann dann eine Gefahrenquelle sein, wenn das Gerät unachtsam gehandhabt wird und so Strahlung auf Bereiche gelangt, die sie nicht erreichen soll, wie zum Beispiel das Auge.

Der Erfindung liegt die Aufgabe zugrunde, Vorrichtungen für medizinische und/oder kosmetische Strahlungsanwendungen bereitzustellen, bei denen die Sicherheit erhöht ist.

Hierzu schlägt die Erfindung Einrichtungen vor zum Feststellen, ob ein der Haut zugekehrtes Bauteil der Vorrichtung einen vorgegebenen Abstand von der Haut hat, und zum Steuern der Strahlungsabgabe der Vorrichtung in Abhängigkeit von der Feststellung.

Damit erreicht die Erfindung in einfacher Weise eine erhöhte Sicherheit bei der Handhabung gattungsgemäßer Geräte. Mit solchen Sicherheitseinrichtungen ausgestattete Behandlungsgeräte können auch weniger geschultem Personal, wie dem Patienten selbst auch zur häuslichen Anwendung in die Hand gegeben werden.

Für die Einrichtungen zum Feststellen, ob ein der Haut zugekehrtes Bauteil der Vorrichtung einen vorgegebenen Abstand von der Haut hat, der geringer ist als ein vorgegebener Maximalabstand, bei dem die Sicherheit beeinträchtigt ist, schlägt die Erfindung unterschiedliche Mittel vor: Besonders geeignet für diesen Zweck sind Kraftsensoren, Lichtsensoren, elektrische Messeinrichtungen, und auch z.B. IR-Detektoren, welche die von der Haut reflektierte IR-Strahlung erfassen und auswerten.

Die Erfindung eignet sich besonders für kompakte Handgeräte.

Das genannte, der Haut zugekehrte Bauteil der Vorrichtung, welches den vorgegebenen Abstand von der Haut haben muss, kann zum Beispiel die lichtemittierende Fläche des Gerätes sein, oder auch ein anderes Bauteil, welches bei bestimmungsgemäßem Gebrauch der Vorrichtung den geringsten Abstand von der Haut hat.

Nachfolgend werden Ausführungsbeispiele der Erfindung anhand der Zeichnung näher beschrieben. Es zeigt bzw. zeigen:
- Figur 1: schematisch einen Schnitt durch ein erstes Ausführungsbeispiel einer Vorrichtung für die Bestrahlung von Haut;
- Figur 2: schematisch eine Ausführung einer solchen Vorrichtung mit einem Kraftsensor;
- Figuren 3, 4: Ausführungsbeispiele der Anordnung von Kraftsensoren für eine Vorrichtung gemäß Figur 1;
- Figur 5: schematisch eine Schaltungsanordnung für eine Vorrichtung gemäß Figur 1, bei der ein Hautwiderstand gemessen wird;
- Figuren 6, 7: Ausführungsbeispiele für Ausgestaltungen einer Widerstandsmessung bei einer Vorrichtung gemäß Figur 1;
- Figur 8: ein Ausführungsbeispiel einer Vorrichtung gemäß Figur 1 mit einem Lichtsender und -empfänger; und
- Figur 9: ein Ausführungsbeispiel für eine Vorrichtung gemäß Figur 1 mit einem IR-Detektor als Einrichtung zum Festellen, ob ein der Haut zugekehrtes Bauteil der Vorrichtung einen vorgegebenen Abstand von der Haut hat.

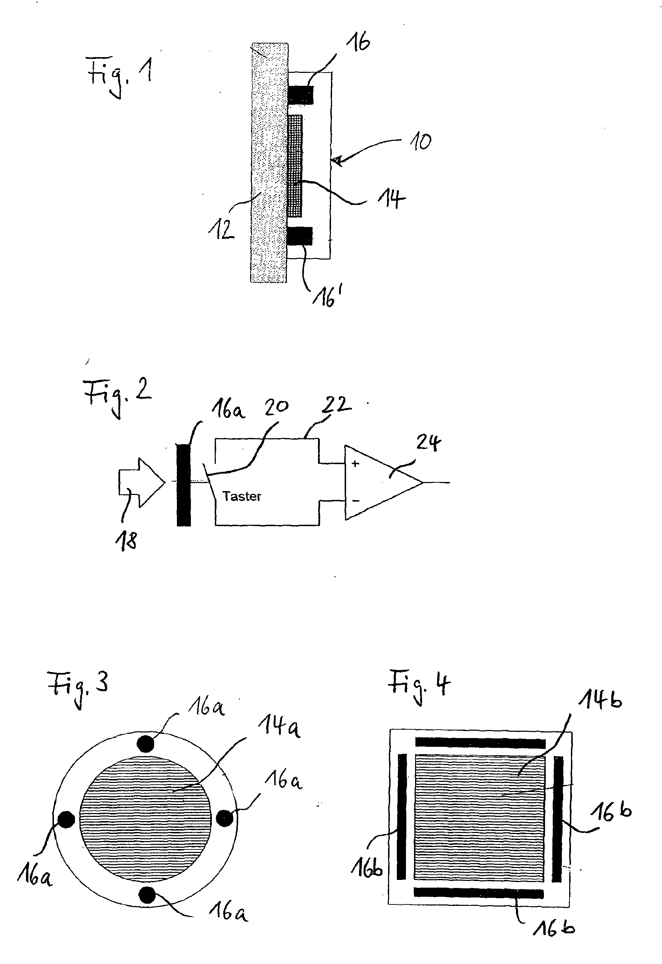

Figur 1 zeigt schematisch eine Vorrichtung 10 für die Bestrahlung von Haut 12 mit elektromagnetischer Strahlung. Die Strahlung wird homogen über eine Behandlungsfläche 14 auf die Haut 12 aufgebracht. Einzelheiten der Strahlungsquelle und gegebenenfalls optischer Mittel zur Lenkung der Strahlung sind nicht dargestellt, da sie dem Stand der Technik entsprechen können.

Einrichtungen 16, 16' sind vorgesehen zum Feststellen, ob ein der Haut 12 zugekehrtes Bauteil der Vorrichtung einen vorgegebenen Abstand von der Haut hat. Dieses Bauteil ist beim in Figur 1 dargestellten Ausführungsbeispiel die Behandlungsfläche 14 der Strahlung, d.h. die Fläche, aus welcher die Strahlung in Richtung auf die Haut austritt.

Dann, wenn die Einrichtungen 16, 16' anzeigen, dass der Abstand zwischen der Behandlungsfläche 14, also dem Bereich, in dem die Strahlung aus der Vorrichtung 10 in Richtung auf die Haut austritt, und der Haut 12 geringer ist als ein vorgegebener Wert, der so bemessen ist, dass er unbeabsichtigte Wirkungen der Strahlung verhindert, spricht die Einrichtung 16, 16' an und erlaubt eine Strahlungsabgabe der Vorrichtung 10 so, dass nur dann Strahlung abgegeben wird, wenn die Vorrichtung 10 nahe genug an der Haut 12 liegt.

Figur 2 zeigt ein erstes Ausführungsbeispiel, bei dem die Einrichtung zum Feststellen des Abstandes zwischen der Vorrichtung 10 und der Haut 12 durch einen Kraftsensor 16a verwirklicht ist. Die Vorrichtung 10 wird in Anlage an die Haut 12 gebracht und dabei übt die Haut eine Reaktionskraft auf den Kraftsensor 16a aus, welcher einen Tastschalter 20 in Abhängigkeit von der Kraft betätigt. Zum Beispiel kann der Sensor 16a mit dem Tastschalter 20 gegen die Richtung der Kraft 18 vorgespannt sein, und nur dann, wenn die Kraft 18 die Vorspannung überwindet, schaltet der Tastschalter 20 und über einen elektrischen Schaltkreis 22, einen Verstärker 24 etc. wird das Schaltsignal dahingehend ausgewertet, dass die Abgabe von elektromagnetischer Strahlung aus der Vorrichtung 10 in Richtung auf die Haut 12 freigegeben ist.

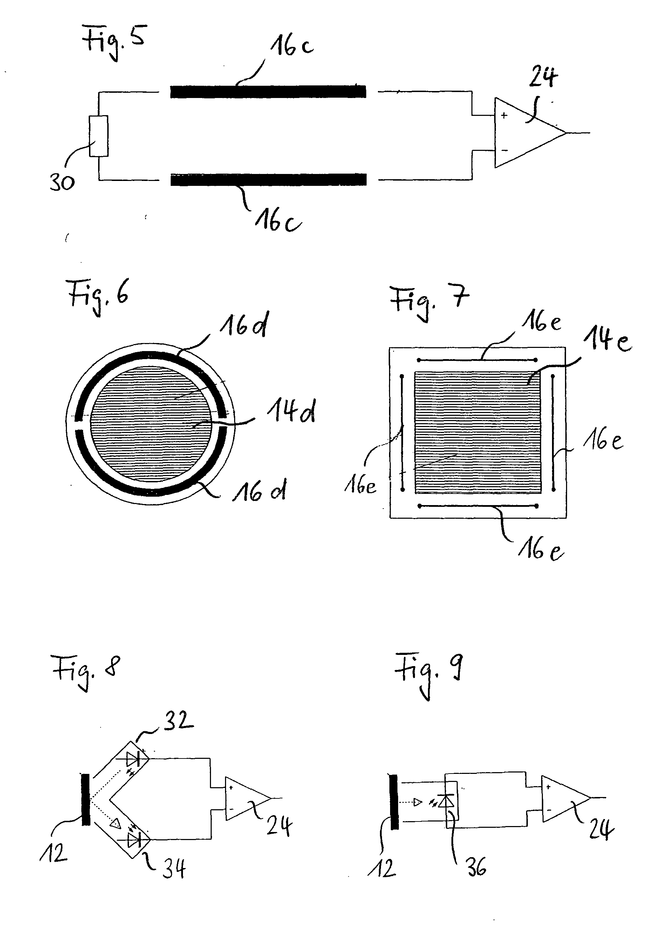

Figur 3 zeigt nähere Einzelheiten der in Figur 2 schematisch dargestellten Anordnung mit vier punktförmigen Kraftsensoren 16a, die über den Umfang um die Behandlungsfläche 14a herum angeordnet sind. Die elektrische Schaltung kann dann so ausgelegt werden, dass alle vier Kraftsensoren 16a einen hinreichenden Druck erfahren müssen, damit die Abgabe von Strahlung aus der Vorrichtung 10 zugelassen ist. Dies gewährleistet, dass die Vorrichtung 10 gleichmäßig und dicht auf die Haut aufgesetzt werden muss, um überhaupt die Abgabe von Behandlungsstrahlung zu ermöglichen.

Figur 4 zeigt eine Abwandlung des Ausführungsbeispieles gemäß Figur 3 mit einer rechteckförmigen Behandlungsfläche 14b und streifenförmigen Kraftsensoren 16b, ansonsten kann die Schaltung und Auswertung analog dem Beispiel gemäß Figur 3 erfolgen.

Figur 5 zeigt schematisch ein anderes Ausführungsbeispiel, bei dem die Einrichtung zum Feststellen, ob die Vorrichtung 10 hinreichend nahe an der Haut 12 liegt, Kontakte 16c aufweist zum Messen eines Hautwiderstandes 30. Eine unschädliche Spannung (Schutz-Kleinspannung) wird zwischen die Kontakte 16c angelegt und dann, wenn die Kontakte hinreichenden Hautkontakt haben, fließt ein Strom über die Haut, der über einen Verstärker 24 verstärkt und dann analog wie oben beschrieben ausgewertet wird, um die Abgabe von Strahlung zu steuern.

Die Figuren 6 und 7 zeigen Ausführungsbeispiele für geeignete Kontakte. Bei einer runden Kontaktfläche 14d eignen sich zum Beispiel halbkreisförmige Kontaktstreifen 16d, zwischen den die elektrische Spannung angelegt ist. Dann, wenn beide Kontaktstreifen 16d hinreichend satt an der zu behandelnden Haut anliegen, ist der insgesamt durch den Hautwiderstand und die Kontakte gegebene Widerstand gering genug, um einen Stromfluss zu erlauben, der anzeigt, dass der Kontakt insgesamt zufriedenstellend ist und die Abgabe von Behandlungsstrahlung freigegeben werden kann. Figur 7 zeigt analog eine quadratförmige Behandlungsfläche 14c und entsprechend auf vier Seiten des Quadrates jeweils einander gegenüberliegende Kontaktstreifenpaare 16e. Jeweils zwischen gegenüberliegenden Kontaktstreifen wird eine Spannung angelegt und dann, wenn alle Kontaktstreifen 16e hinreichend satt an der zu behandelnden Haut aufliegen, zeigt die Messung einen hinreichend geringen Widerstand für die Stromflüsse an, sodass die Strahlung freigegeben werden kann.

Figur 8 zeigt ein weiteres Ausführungsbeispiel, bei dem ein Lichtsender 32, zum Beispiel eine LED, Strahlung in Richtung auf die zu behandelnde Haut 12 emittiert, die dort reflektiert wird und zu einem Lichtsensor 34, zum Beispiel einer Fotodiode, gelangt und dort ausgewertet wird. Dann, wenn die Vorrichtung 10 hinreichend nahe an der Haut 12 ist, wird genügend Strahlung reflektiert, um den Sensor 34 und die Auswerteschaltung 24 im Sinne einer Freigabe der Behandlungsstrahlung ansprechen zu lassen. Dieses Ausführungsbeispiel kann dahingehend abgewandelt werden, dass ohne zusätzliche gesonderte Strahlungsquelle die Behandlungsstrahlung selbst dahingehend ausgewertet wird, ob ein hinreichender Anteil dieser Strahlung an der Hautoberfläche reflektiert wird um damit anzuzeigen, dass die Vorrichtung nahe genug an der Haut ist.

Figur 9 zeigt ein weiteres Ausführungsbeispiel einer in einer Vorrichtung 10 gemäß Figur 1 verwendbaren Einrichtung zum Feststellen, ob die Vorrichtung der Haut nahe genug ist. Beim Ausführungsbeispiel gemäß Figur 9 ist ein Detektor für IR-Strahlung, zum Beispiel eine IR-Fotodiode 36, vorgesehen, um von der Haut 12 abgestrahlte Wärmestrahlung zu detektieren und entsprechend eine elektrische Schaltung so zu steuern, dass dann, wenn hinreichend Wärmestrahlung empfangen wird, dies als Maß dafür gilt, dass die Vorrichtung nahe genug an der Haut ist und so die Behandlungsstrahlung abgegeben werden kann.

## Patentansprüche

1. Vorrichtung für die Bestrahlung von Haut, **gekennzeichnet durch** Einrichtungen (16) zum Feststellen, ob ein der Haut (12) zugekehrtes Bauteil (14) der Vorrichtung (10) einen vorgegebenen Abstand von der Haut hat, und zum Steuern der Strahlungsabgabe der Vorrichtung in Abhängigkeit von der Feststellung.

2. Vorrichtung nach Anspruch 1, **dadurch gekennzeichnet, dass** die Einrichtungen einen Kraftsensor (16a) aufweisen.

3. Vorrichtung nach Anspruch 1, **dadurch gekennzeichnet, dass** die Einrichtungen einen Lichtsensor (34) aufweisen.

4. Vorrichtung nach Anspruch 3, **dadurch gekennzeichnet, dass** dem Lichtsensor (34) ein gesonderter, von der Bestrahlungsquelle der Vorrichtung unabhängiger Lichtsender (32) zugeordnet ist.

5. Vorrichtung nach Anspruch 1, **dadurch gekennzeichnet, dass** die Einrichtungen Mittel (16c, 16d, 16e) aufweisen zum Messen einer elektrischen Größe.

6. Vorrichtung nach Anspruch 5, **gekennzeichnet durch** Mittel zum Messen eines elektrischen Widerstandes.

7. Vorrichtung nach Anspruch 5, **gekennzeichnet durch** Mittel zum Messen eines elektrischen Stromes.

8. Vorrichtung nach Anspruch 5, **gekennzeichnet durch** Mittel zum Messen eines elektrischen Spannungsabfalls.

9. Vorrichtung nach Anspruch 1, **dadurch gekennzeichnet, dass** die Einrichtungen Mittel (36) aufweisen zum Messen von IR-Strahlung.

10. Vorrichtung nach einem der vorhergehenden Ansprüche als Handgerät.
